Europäisches Patentamt

European Patent Office

Office européen des brevets

(11) Publication number: **0 265 964**
**A2**

(12) # EUROPEAN PATENT APPLICATION

(21) Application number: 87115984.4

(22) Date of filing: 30.10.87

(51) Int. Cl.⁴: **C07C 51/353** , C07C 67/343 ,
B01J 23/18 , C07C 57/04 ,
C07C 69/54

(30) Priority: 31.10.86 JP 258460/86
01.07.87 JP 162252/87

(43) Date of publication of application:
04.05.88 Bulletin 88/18

(84) Designated Contracting States:
DE FR GB

(71) Applicant: MITSUBISHI GAS CHEMICAL
COMPANY, INC.
5-2, Marunouchi 2-chome Chiyoda-Ku
Tokyo(JP)

(72) Inventor: Kida, Koichi
18-1, Tsukushino 1-chome
Niigata-shi Niigata-ken(JP)
Inventor: Ebata, Shuji
1-50, Matsuzono 1-chome
Niigata-shi Niigata-ken(JP)
Inventor: Yamamoto, Yasuo
4-15, Koudo 2-chome
Niigata-shi Niigata-ken(JP)

(74) Representative: Vossius & Partner
Siebertstrasse 4 P.O. Box 86 07 67
D-8000 München 86(DE)

(54) Process for preparing alpha,beta-unsaturated aliphatic monocarboxylic acids or esters thereof.

(57) A process is disclosed for producing stably and over a long period of time, $\alpha$ , $\beta$ -unsaturated aliphatic monocarboxylic acids or esters thereof from saturated aliphatic monocarboxylic acids or esters thereof and formaldehyde or derivatives thereof by aldol condensation-dehydration reaction. The outstanding feature of the process resides in the catalyst employed. The catalyst comprises a silica carrier carrying thereon at least one antimony compound and at least one alkali metal compound. The catalyst is free from sintering and has a long life. Further advantages, e.g., a high catalytic activity and high selectivity, can be obtained by the use of a silica carrier with an alumina content of not more than 500 ppm.

EP 0 265 964 A2

## Process for Preparing $\alpha$, $\beta$-Unsaturated Aliphatic Monocarboxylic Acids or Esters Thereof

This invention relates to a process for preparing an $\alpha$, $\beta$-unsaturated aliphatic monocarboxylic acid or an ester thereof from a saturated aliphatic monocarboxylic acid or an ester thereof and formaldehyde or a derivative thereof, said $\alpha$, $\beta$-unsaturated aliphatic monocarboxylic acid or ester thereof having one incremental carbon atom to said saturated aliphatic monocarboxylic acid or ester thereof. $\alpha$, $\beta$-Unsaturated aliphatic monocarboxylic acids and esters thereof are very useful substances as raw materials of various kinds of plastics and the like.

It is known that an $\alpha$, $\beta$-unsaturated aliphatic monocarboxylic acid or an ester thereof can be prepared from a saturated aliphatic monocarboxylic acid or an ester thereof (hereinafter abbreviated occasionally to "saturated acid") and formaldehyde or derivative thereof by aldol condensation-dehydration reaction represented by the following reaction scheme:

$$R-CH_2-COOR' + HCHO \rightarrow R-C(=CH_2)-COOR' + H_2O$$

in which R and R' are independently selected from a hydrogen atom and alkyl groups.

There have been proposed various silica type catalysts carrying alkali metal compounds thereon. For instance, Japanese Patent Publication No. Sho 57 (1982) -40130 discloses the use of alkali metal compounds dispersed on a solid carrier and Japanese Patent Publication No. Sho 45 (970)-21928 discloses the use of alkali metal or alkaline earth metal hydroxides on alumino-silicate or silica gel to prepare an $\alpha$, $\beta$-unsaturated aliphatic monocarboxylic acid or an ester thereof (hereinafter abbreviated occasionally to "unsaturated acid").

In United States Patent No. 393388 (1976), a basic catalyst composed of pyrogenic silica is used and in Japanese Patent Laid-open No. Sho 61(1986)-15737 and Japanese Patent Laid-open No. Sho 61(1986)-15857, catalysts carrying a metal of group I or II in the Periodic Table of Elements on silica are employed to effect this reaction.

Conventional silica type catalysts carrying an alkali metal compound thereon have an insufficient catalytic activity, i.e., they give only a low conversion rate and generally attain only a low rate of selectivity to an unsaturated acid which is the object compound. Raising the reaction temperature in order to attain a high rate of conversion secondarily causes decomposition or polymerization of the unsaturated acid, thus giving a low selectivity and ultimate yield of the unsaturated acid. Further, in order to attain a high selectivity on the basis of formaldehyde, a great excess amount of the saturated acid must be used.

Furthermore, the alkali metal carried on the catalyst tends to dissipate or flow out from the surface of the catalyst during long periods of operation. Another problem is that the presence of alkali metal is liable to cause structural changes of the catalyst, i.e., so-called sintering, such as expansion of the pore size on the catalyst surface. These phenomena bring about a decrease in catalyst activity and selectivity during the operation, and result in a shorter catalyst life.

We have made intensive studies in order to improve insufficient yield and short catalyst life when using such silica catalysts carrying alkali metals thereon. We found that remarkable effects were obtained by promoting the reaction, by prohibiting the flow-out of alkali metal, and by preventing the occurrence of sintering phenomena by adding an antimony compound to said catalyst as a third component.

It was also found that the use of a silica carrier with a lower alumina content and with an antimony compound added thereto brings about a synergetic effect in increasing selectivity of the aldol condensation reaction.

Accordingly, the first object of this invention is to provide a process for preparing advantageously an $\alpha$,$\beta$-unsaturated aliphatic monocarboxylic acid or an ester thereof on an industrial scale from a saturated aliphatic monocarboxylic acid or an ester thereof, said $\alpha$,$\beta$-unsaturated aliphatic monocarboxylic acid or ester thereof having one incremental carbon atom to said saturated aliphatic monocarboxylic acid or ester thereof.

The second object of this invention is to provide a process for preparing an $\alpha$,$\beta$-unsaturated aliphatic monocarboxylic acid or an ester thereof having one incremental carbon atom to the starting material, i.e., a saturated aliphatic monocarboxylic acid or an ester thereof, which is stable for a long period of time.

The third object of this invention is to provide a process for carrying out the above reaction in a high yield and high selectivity.

The forth object of this invention is to provide a catalyst composition to be used in practicing the above process.

Other objects of the invention will be apparent to those skilled in the art from the description of the invention hereinbelow.

Thus, the gist of this invention resides in a process for preparing an $\alpha$, $\beta$ -unsaturated aliphatic monocarboxylic acid or an ester thereof from a saturated aliphatic monocarboxylic acid or an ester thereof, said $\alpha$, $\beta$ -unsaturated aliphatic monocarboxylic acid or ester thereof having one incremental carbon atom to said saturated aliphatic monocarboxylic acid or ester thereof, which comprises reacting said saturated aliphatic monocarboxylic acid or ester thereof with formaldehyde or a derivative thereof in the vapor phase and in the presence of a catalyst comprising a silica carrier carrying thereon at least one antimony compound and at least one alkali metal compound.

In general, the silica used as a carrier for the catalyst in this invention may be a silica gel or a silica sol which is generally prepared from sodium silicate or potassium silicate.

It is desirable, however, that the silica carrier employed in the present invention has a high purity. Generally, the presence of a metal ion is not desirable. Although the alkali metal compound is to be intentionally carried on the catalyst during its preparation, it is desirable to keep the content of the alkali metal compound (originally contained in silica) as low as possible, because its presence is not favorable in adjusting the catalyst performance. It is also desirable to keep the content of other co-existing metal compounds such as alkaline earth metal compounds and transition metal compounds at less than 500 ppm. Among these metal compounds, alumina is the one having a serious adverse effect on the reaction, and thus it is necessary, in order to obtain the catalyst with a particularly high performance, to keep the alumina ($Al_2O_3$) content at less than 500 ppm. Where silica sol is used as the silica source, the alumina ($Al_2O_3$) content of the catalyst silica carrier which has been converted to silica gel should be less than 500 ppm.

The alumina content of a silica gel used for a catalyst is generally approximately 0.1% (1000 ppm) by weight. However, recent improvements in the metal ion elimination step by means of ion exchange in the conventional silica gel manufacturing process and the development of a process for preparing a silica gel by hydrolysis of an organosilicon compound such as tetraalkoxysilane have made it possible to prepare a silica gel with the alumina content of less than 500 ppm and a silica sol which can give a silica gel with the alumina content of less than 500 ppm at a dried state. In this invention, it is preferable, particularly in order to prepare a high performance catalyst, to employ such silica gel or silica sol having a low alumina content.

The alkali metal compound to be carried on silica may be a salt, a hydroxide, or an alcoholate of at least one alkali metal belonging to the group I of Mendelejeff's Periodic Table of Elements such as sodium, potassium, rubidium, and cesium. Particularly preferred metals are potassium, rubidium, and cesium. The amount of the alkali metal compound to be carried or supported on silica gel may be determined taking the catalyst performance on the reaction into account, but it is usually within the range of 0.5 - 20% by weight, preferably 1.0 - 10% by weight, based on the total amount of silica gel and the third component.

The antimony compound to be added as the third component may preferably be antimony pentachloride, antimony pentafluoride, antimony pentasulfide, antimony potassium tartarate, antimony tribromide, antimony trichloride, antimony trioxide, antimony pentoxide, antimony triethoxide and the like. The particularly preferred antimony compound is antimony pentoxide ($Sb_2O_5$).

The amount of the third component to be added may be 0.2 - 30% by weight, preferably 0.5 - 20% by weight, as $Sb_2O_5$ based on the amount of silica gel. An amount less than the above range gives only a poor effect on preventing sintering, while an amount more than the above range seriously impairs the catalyst performance in the reaction. The actual amount of the antimony compound to be added can be determined depending on the kind of the alkali metal to be employed, the amount of the alkali metal compound to be carried on the catalyst, the kind of the raw material silica, and the kind of the third component. In general, a greater amount of the third component should be added when (i) the third component to be added is an oxide rather than a salt, (ii) the raw material silica is a silica sol rather than a silica gel, (iii) the amount of an alkali metal compound carried on the catalyst is large rather than small, and (iv) the alkali metal used has a low atomic weight rather than a high one.

There are various methods for adding antimony but the differences in the method to be employed in the catalyst preparation do not give any effect on the spirit of this invention. Generally acceptable methods are, for example, the method of gelling a silica sol after the addition of an antimony salt or oxide sol; the method of dissolving or suspending an antimony salt into a water-soluble silicate such as sodium silicate and potassium silicate, and then neutralizing and precipitating the mixture by the addition of a precipitating agent; and the method of impregnating a water-soluble antimony salt into a silica gel.

The addition of an alkali metal compound may be carried out according to any method known to the art. Examples of desirable methods are the method of impregnating a soluble alkali metal salt to a silica gel which has been added with an antimony compound, and the method of replacing silanol group on the surface of silica with an alkali metal by means of ion exchange methods. The method of adding an alkali metal compound simultaneously with an antimony compound may also be employed. The catalyst onto which an antimony compound has been added and an alkali metal compound has been carried, is dried or calcined according to a known method.

Characteristics of the catalyst when dried and calcined as required, particularly its pore surface area and the value of the pore size distribution peak, may vary depending on the method by which the catalyst has been prepared, though pore surface area is preferably in the range of 20 -600 m2/g, more preferably 50 - 600 m2/g, and the value of the pore size distribution peak is preferably 40 - 1000 Å more preferably 60 -500 Å. This is because a catalyst with a smaller value of the pore size distribution peak and a greater surface area causes more side reactions, and a catalyst with a smaller surface area does not have a sufficient activity.

The saturated aliphatic monocarboxylic acid or the ester thereof, the raw material, is a compound represented by formula R-CH$_2$-COOR', in which R represents a hydrogen atom or an alkyl group with 1 - 3 carbon atoms and R' represents a hydrogen atom or an alkyl group with 1-5 carbon atoms. Examples of the carboxylic acid satisfying this formula are acetic acid, propionic acid, butyric acid, valeric acid and iso-valeric acid, and examples of esters satisfying the formula are methyl, ethyl, n-propyl, isopropyl, n-butyl, sec-butyl, tert-butyl, iso-butyl and n-pentyl esters and the like of the carboxylic acid. Among them, commercially important compounds are acetic acid, propionic acid, methyl acetate, ethyl acetate, methyl propionate, and ethylpropionate. Acrylic acid and esters thereof are produced from acetic acid and esters thereof, and methacrylic acid and esters thereof are produced from propionic acid and esters thereof, respectively.

As formaldehyde or derivatives thereof, there are used, beside formaldehyde itself, compounds producing formaldehyde by decomposition thereof preferably formalin, a polymeric compound of formaldehyde, like trioxan and paraformaldehyde, a formal like dimethoxy methane, or a hemi-formal.

The saturated acid and formaldehyde or derivative thereof (hereinafter abbreviated occasionally to formaldehyde) used as the raw materials are fed to the reaction system as a mixed gas. The molar ratio of saturated acid vs. formaldehyde (saturated acid/formaldehyde) is 10/1 -1/3, preferably 5/1 - 1/2, and more preferably 3/1 - 1/2. The space velocity on catalyst (WHSV) based on the total feed is in a range of 0.1 - 30 g/hr/g-cat., and preferably 1.0 - 10 g/hr/g-cat. The reaction temperature is 200 -500°C, and preferably 250 - 400°C. Although there is no specific limitation on the reaction pressure, the generally employed pressure is preferably less than 5 kg/cm2G.

The reaction is carried out continuously except for special cases. There is no restriction with respect to the types of reactor bed. A fixed-bed continuous reactor is employed in general, though a moving-bed or fluidized-bed reactor may also be adopted. The form or shape of the catalyst may be varied depending on the types of the reactor. The size of the catalyst is usually larger than 100 mesh (sieve opening: 0.147 mm) when using a fixed-bed type reactor, and pills with a diameter of more than 1 mm or pellets with a diameter of more than 1 mm and a length of more than 1 mm are employed for commercial application.

During the course of aldol condensation reaction, the addition of an alkali metal compound to the silica gel carrier may cause the so-called sintering phenomena such as decrease in pore surface area, reduction of pore volume and increase in the value of the pore size distribution peak.

Although there may be conceivable various third components for preventing the sintering from taking place, we have found that antimony compounds possess the desired properties without impairing the principal purpose of the reaction of this invention. Although the detailed mechanism of the effect of an antimony compound has not been elucidated yet, it may be possible that an antimony compound is incorporated into, or particles of an antimony compound may be commingled with the substrate structure of silica, thereby restricting silica particles from agglomeration.

Use of a catalyst comprising a silica carrier with an alumina content of not more than 500 ppm and with an alkali metal compound carried thereon can increase the selectivity to the unsaturated acid but decrease the catalytic activity and thus reduces the rate of conversion of the raw material saturated acid, and its use is also liable to cause sintering. Addition of an antimony compound, however, can promote its catalytic activity, and at the same time improve the selectivity to the unsaturated acid and the catalyst life. From these facts it will be understood that the catalyst comprising a silica carrier with an alkali metal compound carried thereon can exhibit its catalytic activity more efficiently by the combination of a silica having a low alumina content and an antimony compound.

Although the reasons are not elucidated in detail, it is presumed that an acid-base balance and an acid-base distribution on the catalyst surface may be brought to a state suitable for the reaction due to the decrease in alumina content which will reduce the acidity of silica, the presence of an alkali metal compound which will provide basicity, and the presence of an antimony compound which can be converted by calcination into antimony pentoxide which will provide acidity.

As illustrated above, unexpectedly good results were obtained by incorporating an antimony compound to a catalyst comprising a silica gel carrier with a low alumina content carrying an alkali metal compound thereon. The invention provides a great industrial advantage by remarkably increasing the yield of unsaturated acid and improving the catalyst life.

The invention is hereinbelow described by way of examples, which shall by no means be construed as limiting this invention.

In the description hereinbelow the catalyst performance on the reaction is defined by the following formulae:

$$\text{Saturated Acid Conversion Rate (\%)} = \frac{\text{Moles of reacted saturated acid}}{\text{Moles of charged saturated acid}} \times 100$$

$$\text{Unsaturated Acid Selectivity (saturated acid base) (\%)} = \frac{\text{Moles of produced unsaturated acid}}{\text{Moles of reacted saturated acid}} \times 100$$

$$\text{Unsaturated Acid Yield (saturated acid base) (\%)} = \frac{\text{Moles of produced unsaturated acid}}{\text{Moles of charged saturated acid}} \times 100$$

$$\text{Formaldehyde}^1 \text{ Conversion Rate (\%)} = \frac{\text{Moles of reacted formaldehyde}^1}{\text{Moles of charged formaldehyde}^1} \times 100$$

$$\text{Unsaturated Acid Selectivity (formaldehyde}^1 \text{ base) (\%)} = \frac{\text{Moles of produced unsaturated acid}}{\text{Moles of reacted formaldehyde}^1} \times 100$$

$$\text{Unsaturated Acid Yield (formaldehyde}^1 \text{ base) (\%)} = \frac{\text{Moles of produced unsaturated acid}}{\text{Moles of charged formaldehyde}^1} \times 100$$

$^1$: including formaldehyde derivatives, in the case of formaldehyde derivatives these are recalculated as formaldehyde

As a measure for designating changes in the catalysts surface structure, i.e., the sintering, the value of the pore size distribution peak of the catalyst was measured using an automatic pore size distribution tester (AUTOPORE® Model 9200, max. pressure 60,000 psig; manufactured by Micrometrics Co.). The catalyst whose value of the pore size distribution peak after it has been used shifts to a greater extent to a larger pore size side as compared to that of the original catalyst is deemed to undergo a higher degree of sintering.

In examples below, unless specifically designated otherwise, "%" and "part" designate "% by weight" and "part by weight", respectively.

## Example 1

(Preparation of the catalyst)

One hundred (100) parts of a low alumina silica gel (ST-N-30, $SiO_2$ content: 30.6%; product of Nissan Chemical Industries, Ltd.) and 7.5 parts of an antimony sol (FER-6N, Lot No. 503Z4, $Sb_2O_5$ content: 16.8%, particle size 10 - 30 $m\mu$ ; product of Nissan Chemical Industries, Ltd.) were mixed together. An aqueous ammonia was added to the mixture to make it to almost neutral and further a 10% aqueous solution of ammonium nitrate was added to the mixture while vigorously agitating it for the gellation to take place. The gelled material was charged to an grinding mill and was ground for 2 hours. The ground material was put into a flask equipped with a reflux condenser and heated under agitation by means of a mantle heater for 1.5 hours. The resulting slurry was transferred to a porcelain dish and most of the water contained therein was removed by vaporization in an electronic range. Then the material was transferred to an alumina crucible and calcined in the air at 500°C for 4 hours.

The calcined product was ground in a mortar to adjust the particle size to 10 - 20 meshes (1.65 to 0.83 mm sieve opening). The content of alumina ($Al_2O_3$) in the carrier thus obtained was 320 ppm. The carrier was added with 56 parts of 4% aqueous solution of cesium nitrate and transferred to a rotary evaporator to distill off water by an aspirator in vacuum while heating it in a water bath at 80°C. The product was dried overnight at 130°C and calcined at 500°C for 4 hours. The product thus prepared was served as the catalyst. The cesium metal content of the catalyst was about 5.0% based on the carrier and its antimony content was about 4% as $Sb_2O_5$.

(Test for Initial Catalyst Activity)

Two Pyrex® glass tube reactors with internal diameter of approximately 13 mm were used for the test. They were so designed as to be heated separately by external heaters and connected in series so as to use the front tube as a decomposition reactor for producing formaldehyde gas and the other tube as the reactor for aldol condensation.

As raw materials, propionic acid was used as the saturated acid and trioxan was used as the formaldehyde source. A mixed liquid with a propionic acid/trioxan (calculated as formaldehyde) molar ratio of 3/2 was fed to the trioxan decomposition reactor at 20 g/hr by means of a metering pump.

The trioxan decomposition reactor was packed with 20 g of catalyst consisting of silica-alumina (N-631; product of Nikki Kagaku Co., Ltd.) carrying 15% phosphoric acid thereon. Trioxan was quantitatively converted into formaldehyde at about 200°C, which was introduced to the aldol condensation reactor together with gaseous propionic acid as the raw materials.

The aldol condensation reactor was packed with 5 g (11.4 ml) of the catalyst prepared as described above and the reactions were carried out at temperatures of 320°C, 300°C and 340°C, respectively. The feed rate of the raw material per unit weight-hour of the catalyst (WHSV) was 4.0 g/hr.g-cat. and the gas hourly space velocity (GHSV) was 700 $\ell$/hr.$\ell$-cat.

The reaction product was absorbed in ice-cooled water and subjected to analysis by means of gas chromatography.

(Catalyst Life Test)

The above-mentioned reaction was carried out under the same conditions as above and at a temperature of 330°C for net 16 days. Decoking was performed every two days to remove the deposited coke by burning off, as the reaction involves coke deposition during a continuous operation. Decoking was conducted by stopping the feed of the raw material and feeding nitrogen gas with a small amount of air mixed therewith (oxygen content: 2 - 10 volume %) while maintaining the temperature at 400°C.

The value of the pore size distribution peak and the cesium content of the catalyst, before loading the catalyst and after the termination of the catalyst life test, were measured in order to observe the catalyst life. The results are shown in Table 1.

Example 2

This example is given not for the purpose of illustrating the practice of this invention but for the purpose of <u>comparison</u>.

(Preparation of Catalyst)

One hundred (100) parts of a silica sol (Snowtex®30, $SiO_2$ content: 30%; product of Nissan Chemical Industries, Ltd.) was gelled following the same procedure as in Example 1, except that addition of antimony sol was omitted. The alumina ($Al_2O_3$) content of this silica gel was 950 ppm.

Cesium nitrate was carried on the silica gel according to the same manner as in Example 1 to prepare a catalyst with a cesium metal content of about 5%.

(Catalyst Activity Test)

The catalyst activity was tested in the same manner as in Example 1, except that the reaction temperatures of 330°C, 340°C and 350°C were adopted, because of a rather lower activity of this catalyst. For attaining the propionic acid conversion rate of the same level as in Example 1, it was necessary to carry out the reaction at a temperature at least 10°C higher than that employed in Example 1.

(Catalyst Life Test)

Operation was conducted at 330°C for net 16 days according to the same manner as in Example 1. The results are shown in Table 1.

In comparison of the catalyst performance of Examples 1 and 2, it can be observed that Example 1 exhibited a superior catalyst performance at the initial activity test and showed a lower decrease with respect to the conversion rate and selectivity in the catalyst life test. Furthermore, because of the smaller changes in the value of pore size distribution peak in Example 1, it can be concluded that the catalyst of Example 1 caused less sintering in an operation extending for a long period of time. The amount of cesium metal flown out from the catalyst surface was also less in the catalyst of Example 1.

7

Table 1

| Examples No. | 1 | | | 2 | | |
|---|---|---|---|---|---|---|
| Reaction Temp. (°C) | 320 | 330 | 340 | 330 | 340 | 350 |
| (Initial Activity Test) Propionic Acid Conversion Rate (%) | 24.3 | 29.5 | 35.2 | 23.7 | 28.8 | 33.7 |
| Methacrylic Acid Selectivity (%) (propionic acid base) | 96.0 | 94.7 | 89.3 | 89.1 | 86.5 | 81.5 |
| Methacrylic Acid Yield (propionic acid base)(%) | 23.3 | 27.9 | 31.4 | 21.1 | 24.9 | 27.5 |
| Formaldehyde Conversion Rate (%) | 39.1 | 49.0 | 57.1 | 40.0 | 49.1 | 60.3 |
| Methacrylic Acid Selectivity (%) (formaldehyde base) | 89.4 | 85.4 | 82.5 | 78.5 | 76.1 | 68.3 |
| Methacrylic Acid Yield (formaldehyde base) (%) | 35.0 | 41.9 | 47.1 | 31.7 | 37.4 | 41.2 |
| (Catalyst Life Test) | 1st day | | 15th day | 1st day | | 15th day |
| Propionic Acid Conversion Rate (%) | 30.5 | | 28.9 | 24.2 | | 17.2 |
| Methacrylic Acid Selectivity (%) (propionic acid base) | 94.0 | | 92.5 | 87.7 | | 86.3 |
| (Catalyst Properties) | Before use | After use | Changes | Before use | After use | Changes |
| The Value of Pore Size Distribution Peak (Å) | 95 | 130 | (35) | 92 | 285 | (193) |
| Cesium Content (wt%) | 4.9 | 4.5 | (0.4) | 5.1 | 1.8 | (2.3) |

Examples 3 - 10

Experiments were conducted according to the same manner as in Examples 1 and 2, with variations, however, with respect to the source of silica, the source and concentration of alkali metal, the concentration of antimony, the sources of saturated acid and formaldehyde, and reaction conditions. The molar ratio of the raw materials was the same as Example 1, i.e., 3/2.

The results obtained are shown in Table 2, in which abbreviations for various raw materials are as follows:

MPA: methyl propionate
PA: propionic acid
AcH: acetic cid
TO: trioxan
PF: paraformaldehyde
MA: methacrylic acid
MMA: methyl methacrylate
AA: acrylic acid;
and the following sources of silica were employed:
Sol-1: Silica sol ST-N-30; product of Nissan Chemical Industries, Ltd.
Sol-2: Snowtex®30; product of Nissan Chemical Industries Ltd.
Sol-3: Silica sol ST-N-15; product of Nissan Chemical Industries, Ltd.
Gel: Gel obtained by alumina elimination treatment of Silica gel ID-57; product of Fuji Davison Chemical

Values for the reaction performance in Table 2 represents those measured at 6 hours following start of the reaction. Where paraformaldehyde was used, the compound was fed to the decomposition reactor as a slurry with propionic acid.

Example 4, which is one of the examples of this invention but employs a high alumina-content silica gel carrier, is given for the purpose of comparison with experiments using a low alumina-content silica gel carriers of Examples 3, 5, 6, and 8 - 10. Example 7 employs a known catalyst not falling into the scope of the claimed invention, but was given for the purpose of comparison.

## Table 2

| Examples No. | 3 | 4 | 5 | 6 | 7 | 8 | 9 | 10 |
|---|---|---|---|---|---|---|---|---|
| **Raw Material** | | | | | | | | |
| Saturated Acid | MPA | MPA | PA | PA | PA | PA | AcH | AcH |
| Formaldehye | TO | TO | TO | PF | PF | TO | TO | TO |
| **Produced Unsaturated Acid** | MMA | MMA | MA | MA | MA | MA | AA | AA |
| **Catalyst** | | | | | | | | |
| Source of Silica | Sol-1 | Sol-2 | Sol-3 | Sol-1 | Sol-1 | Gel | Sol-1 | Gel |
| $Sb_2O_5$ (wt%) | 5 | 5 | 3.5 | 5 | none | 4 | 10 | 8 |
| $Al_2O_3$ (ppm) | 330 | 750 | 80 | 310 | 340 | 240 | 450 | 270 |
| Alkali Metal Kind | Cs | Cs | Cs | K | K | Cs | Cs | Rb |
| Conc. (wt%) | 3.1 | 3.2 | 5.1 | 1.9 | 2.0 | 4.9 | 7.5 | 5.0 |
| Value of Pore Size Distribution Peak (Å) | 105 | 100 | 90 | 95 | 90 | 120 | 115 | 120 |
| **Reaction Conditions** | | | | | | | | |
| Temperature (°C) | 340 | 340 | 330 | 360 | 360 | 340 | 380 | 360 |
| WHSV (g/hr.g-cat.) | 2.0 | 2.0 | 4.0 | 2.0 | 2.0 | 4.0 | 4.0 | 2.0 |
| **Initial Performance** | | | | | | | | |
| Saturated acid Conversion Rate (%) | 30.4 | 27.7 | 29.2 | 32.3 | 27.2 | 28.4 | 31.5 | 28.9 |
| Selectivity (%) (saturated acid base) | 91.4 | 85.1 | 89.7 | 83.5 | 79.3 | 94.1 | 76.4 | 84.4 |
| Yield (%) (saturated acid base) | 27.8 | 23.5 | 26.2 | 27.0 | 21.6 | 26.7 | 24.1 | 24.4 |
| **Catalyst after Reaction** | | | | | | | | |
| Operation Days | 8 | 8 | 16 | 16 | 16 | 16 | 16 | 16 |
| Alkali Metal Conc. (wt%) | 2.9 | 2.2 | 4.8 | 1.7 | 1.2 | 4.5 | 7.1 | 4.5 |
| Value of Pore Size Distribution Peak (Å) | 113 | 210 | 102 | 120 | 270 | 145 | 130 | 135 |

From the above results, it is evident that the Examples 3, 5, 6, and 8 - 10 exhibited an improved conversion rate, selectivity and yield in the initial performance as compared to Examples 4 and 7. Further, it can be understood that the catalyst life was also improved from the fact that decrease in the alkali metal content and change in the value of pore size distribution peak were both small after use in these catalysts.

## Claims

1. A process for preparing an $\alpha$, $\beta$ -unsaturated aliphatic monocarboxylic acid or an ester thereof from a saturated aliphatic monocarboxylic acid or an ester thereof, said $\alpha$, $\beta$ -unsaturated aliphatic monocarboxylic acid or ester thereof having one incremental carbon atom to said saturated aliphatic monocarboxylic acid or ester thereof, which comprises reacting a saturated aliphatic monocarboxylic acid or an ester thereof with formaldehyde or a derivative thereof in the vapor phase and in the presence of a catalyst comprising said silica carrier carrying thereon at least one antimony compound and at least one alkali metal compound.

2. The process according to Claim 1, wherein the content of alumina in said silica carrier is not more than 500 ppm.

3. The process according to Claim 1 or 2, wherein the amount of said antimony compound carried on said silica carrier calculated as $Sb_2O_5$ is from 0.2 to 30% by weight based on the amount of silica.

4. The process according to any one of Claims 1 - 3, wherein the alkali metal of said alkali metal compound is K, Rb, or Cs.

5. The process according to any one of Claims 1 - 4, wherein said saturated aliphatic monocarboxylic acid or ester thereof can be represented by the formula:
$R-CH_2-COOR'$
wherein R represents a hydrogen atom or an alkyl group with 1 - 3 carbon atoms and R' represents a hydrogen atom or an alkyl group with 1 - 5 carbon atoms.

6. The process according to any one of Claims 1 - 5, wherein said derivative of formaldehyde is selected from the group consisting of formalin, trioxan, paraformaldehyde, formal, and hemi-formal.

7. The process according to any one of Claims 1 - 6, wherein said reaction is carried out at a temperature of 200 - 500°C, a pressure of not higher than 5 $Kg/cm^2G$ and a weight hourly space velocity of 0.2 - 30 $hr^{-1}$.

8. The process according to any one of Claims 1 - 7, wherein the molar ratio of said saturated aliphatic monocarboxylic acid or ester thereof/formaldehyde charged to the reaction is 10/1 - 1/3.